# EUROPÄISCHE PATENTSCHRIFT

(11) **EP 1 747 777 B1**
(45) Veröffentlichungstag und Bekanntmachung des Hinweises auf die Patenterteilung: **14.09.2011**
(21) Anmeldenummer: 04821979.4
(22) Anmeldetag: 13.07.2004
(51) Int. Cl.: A61K 31/14, A61P 35/00

(54) **MEDIZINISCHES MITTEL**
MEDICINAL AGENT
PREPARATION MEDICINALE

(30) Priorität: 12.05.2004 RU 2004114297
(43) Veröffentlichungstag der Anmeldung: 31.01.2007
(73) Patentinhaber: Kutushov, Mikhail Vladimirovich, Moscow, 125414 (RU); Germanov, Evgeny Pavlovich, Moscow, 121085 (RU)
(72) Erfinder: KUTUSHOV, Mikhail Vladimirovich, Moscow, 125414 (RU)
(74) Vertreter: Jeck, Anton
(86) Internationale Anmeldenummer: PCT/RU2004/000272
(87) Internationale Veröffentlichungsnummer: WO 2005/110389

(56) Entgegenhaltungen:
- WO-A-01/30408
- DE-A1- 2 709 094
- GB-A- 908 634
- JP-A- 1 067 865
- JP-A- 11 143 407
- JP-A- 57 158 839
- JP-A- 60 104 193
- RU-C1- 2 088 228
- RU-C2- 2 176 505
- RU-C2- 2 195 277
- US-A1- 2002 123 530
- GUILHERME ET AL: "Effect of Molecular Structure on the Performance of Triarylmethane Dyes as Therapeutic Agents for Photochemical Purging of Autologous Bone Marrow Grafts from Residual Tumor Cells" JOURNAL OF PHARMACEUTICAL SCIENCES, Bd. 89, Nr. 1, Januar 2000 (2000-01), Seiten 88-99, XP009088163
- LAVINIA ET AL: "Effect of dye aggregation on triarylmethane-mediated photoinduced damage of hexokinase and DNA" JOURNAL OF PHOTOCHEMISTRY AND PHOTOBIOLOGY, Bd. 67, 2002, Seiten 139-148, XP009088162
- KIYOKO MIYAGI ET AL: "Crystal violet combined with Merocyanine 540 for the ex vivo purging of hematopoietic stem cell grafts" JOURNAL OF PHOTOCHEMISTRY AND PHOTOBIOLOGY, Bd. 70, 2003, Seiten 133-144, XP009088161
- KANDELA ET AL: "Effect of molecular structure on the selective phototoxicity of triarylmethane dyes towards tumor cells" PHOTOCHEMICAL AND PHOTOBIOLOGICAL SCIENCES, Bd. 1, 2002, Seiten 309-314, XP002446479
- ROYBAL ET AL: "HIGH-PERFORMANCE LIQUID CHROMATOGRAPHY OF GENTIAN VIOLET, ITS DEMETHYLATED METABOLITES, LEUCOGENITIAN VIOLET AND METHYLENE BLUE WITH ELECTROCHEMICAL DETECTION" JOURNAL OF CHROMATOGRAPHY, Bd. 467, 1989, Seiten 259-266, XP002446480
- [Online] 1998, Seiten 1 - 2, XP003017554 Gefunden im Internet: <URL:http://stainsfile.info/StainsFile/dyes /42555.htm>
- 'Vrednye veschestva v khimicheskoi promyshlennosti, part 1' ORGANICHESKIE VESCHESTVA, GNTI KHIMICHESKOI LITERATURY, IZD. 4, L. 1963, Seite 639, XP008084973

## Beschreibung

Die Erfindung gehört zum medizinischen Bereich, insbesondere zu antiblastomatösen Heilmitteln, darunter auch zu Präparaten, die auf das Immunsystem einwirken.

Bekannt ist das Präparat Adriamycin, das ein Antibiotikum der Anthracyclin-Gruppe darstellt und eine ausgeprägte antiblastomatöse Wirkung besitzt (s. M.D. Maschkowskij, Arzneimittel, Verlag Medizin, Moskau, 1985, Band 2, S. 460-461).

Das Präparat wird als Hydrochlorid-Lösung ausdrücklich intravenös eingesetzt, da es subkutan Gewebsnekrose verursacht. Darüber hinaus besitzt das Präparat eine kardiotoxische Wirkung und kann Herzschmerzen, eine Herzinsuffizienz und eine Senkung des Blutdrucks verursachen.

Der am nächsten kommende Prototyp ist das Zyclophosphan, das als weißes, kristallines Pulver, wasserlöslich (1:50), leicht alkohollöslich und schwer in isotonischer Natriumchlorid-Lösung löslich, hergestellt wird (z.B. M.D. Maschkowskij, Arzneimittel, Verlag Medizin, Moskau, 1985, Band 2, S. 433-434).

Dieses Präparat besitzt ein breites, antiblastomatöses Wirkungsspektrum und ist dabei ein Immunnosuppressant, bei dessen Einsatz aber Nebenwirkungen beobachtet werden können, wie z. B. Schwindel, Übelkeit und Erbrechen, oft Kopfhaarausfall.

### Einfügung Seite 2a

Der Erfindung wurde die Aufgabe zugrunde gelegt, ein Heilmittel zu finden, das dem Prototyp ähnelt, aber ein breites Wirkungsspektrum ohne Nebenwirkungen besitzt.

Bekannt ist das zur Gruppe der Triaminotriphenylmethan-Farbstoffe gehörende Methylviolett, das Salze mit gefärbten organischen Kationen darstellt (s. z. B. Kurzgefasste, chemische Enzyklopädie, Verlag Sowjetische Enzyklopädie, Moskau, 1967, Band 5, S. 252-253) und das ein Tetra-, Penta- und Hexamethyl-Derivate umfassendes Gemisch aus stickstoffmethyliertem Tri-p-aminotriphenylchlormethan darstellt (s. z.B. StainsFile Methylviolett 2B, 6B, 10B. (http://members.pgonline.com/bryand/-StainsFile/dyes/42535.htm).

Die Strukturformel von Tri-p-aminotriphenylchlormethan hat die Form:

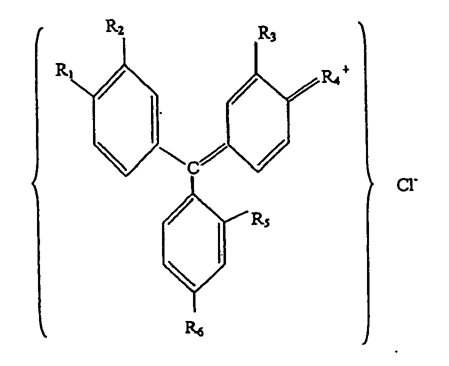

Die Verwendung von Tri-p-aminotriphenylchlormethan-Derivaten im medizinischen Bereich ist aus dem Stand der Technik bekannt, vgl. beispielsweise GUILERME et al., Journal of Pharmaceutical Science, 88-99 (2000), LAVINIA et al., Journal of Photochemistry and Photobiology, 139-148 (2002), KIYOKO MIYAGI et al., Journal of Photochemistry and Photobiology 309-314 (2002), US 2002/123530 A1, WO 01/30408 A, ROYBAL et al., Journal of Chromatography 259-266 (1989), JP 60 104193, JP 01 067865, JP 57 158839 A und JP 11 143407.
in der z. B. R₁ = N(CH₃)₂, R₂ = H, R₃ = H, R₄ = N(CH₃)₂, R₅ = H, R₆= entweder NH₂ oder NHCH₃ oder N(CH₃)₂ ist.

Methylviolett 2B (Tetramethylpararosaniliniumchlorid) ist dabei ein Tetramethyl-Derivat von Tri-p-aminotriphenylchlormethan (in der Strukturformel R₆ = NH₂, Bezeichnungen für R₁, R₂, R₃, R₄, R₅ stimmen mit den oben genannten überein, Summenformel C₂₃H₂₆N₃Cl, Molargewicht 379,94) und stellt ein wasser- und alkohollösliches, feinkörniges, braunes, kristallines Pulver grünlichen Farbtons dar; Methylviolett 6B (Pentamethylpararosaniliniumchlorid) ist ein Pentamethyl-Derivat von Tri-p-aminotriphenylchlormethan (in der Strukturformel R₆ = NHCH₃, Bezeichnungen für R₁, R₂, R₃, R₄, R₅ stimmen mit den oben genannten überein, Summenformel C₂₄H₂₈N₃Cl, Molargewicht 393,97) und stellt ein homogenes, wasserund alkohollösliches, feinkörniges, kristallines Pulver bronze-grünlicher Farbe dar, auch als Grundviolett K bzw. Methylviolett bekannt; Methylviolett 10B (Hexamethylpararosaniliniumchlorid) ist ein Hexamethyl-Derivat von Tri-p-aminotriphenylchlormethan (in der Strukturformel R₆ = N(CH₃)₂, Bezeichnungen für R₁, R₂, R₃, R₄, R₅ stimmen mit den oben genannten überein, Summenformel C₂₅H₃₀N₃Cl, Molargewicht 407,99) und stellt ein wasser- und alkohollösliches, grünes, kristallines Pulver dar, auch als Kristallviolett bzw. Grundviolett 3 bekannt (s. z.B. Katalog Schadstoffe in der Industrie, Teil 1, organische Stoffe, Verlag chemischer Literatur, Ausgabe 4, Leningrad, 1963, S. 638-639, StainsFile Methyl Violet 2B, 6B, 10B. http://members.pgonline.com/-bryand/Stainsfile/dyes/42535.htm sowie Nachschlagewerk für Chemiker, Verlag Chemie, Leningrad, 1967, Band 6, S. 759). Es wird darauf hingewiesen, dass die o.g. Derivate in einigen Quellen als Gentianaviolett bezeichnet werden (s. z. B. StainsFile Methyl Violet 2B, 6B, 10B, http://members.pgonline.com/-bryand/StainsFile/dyes/42535.htm).

Bekannt ist ein Farbstoffgemisch, bestehend aus Methylviolett und Fuchsin unter der Bezeichnung Dahliaviolett, ein wasser- und alkohollösliches, grünes, kristallines Pulver sowie aus Methylviolett und Dextrin unter der Bezeichnung Gentianaviolett, das ein wasserlösliches, grünglänzendes Pulver darstellt (s. z. B. Katalog Schadstoffe in der Industrie, Teil 1, organische Stoffe, Verlag chemischer Literatur, Ausgabe 4, Leningrad, 1963, S. 639).

Diese Farbstoffe werden hauptsächlich in der Industrie eingesetzt, beispielsweise zur Gewebefärbung. Bekannt ist aber der Einsatz von Kristallviolett (Hexamethyl-Derivat von Tri-p-aminotriphenylchlormethan) in der Gynäkologie und Pädiatrie zur Behandlung von Kandidosen (s.http://www.oncology.com).

Bekannt ist Dextrin, das ein Polysaccharid-Gemisch darstellt und in der Gewebefärbung eingesetzt wird (s. N.L. Glinka, Allgemeine Chemie, Verlag Chemie, 1978, S. 494).

Die gestellte Aufgabe wird mittels des immunmodulierenden, antiblastomatösen Arzneimittels nach Anspruch 1 gelöst.

Das Arzneimittel enthält das Tri-p-aminotriphenylchlormethan entweder als Gemisch seiner Tetra- und Pentamethyl-Derivate im Verhältnis von 2,0-98,0 Vol.-% Tetramethyl- und restlichem Pentamethyl-Derivat oder als Gemisch seiner Tetra- und Hexamethyl-Derivate im Verhältnis von 2,0-98,0 Vol.-% Tetramethyl- und restlichem Hexamethyl-Derivat oder als Gemisch dessen Penta- und Hexamethyl-Derivate im Verhältnis von 2,0-98,0 Vol.-% Pentamethyl- und restlichem Hexamethyl-Derivat oder als Gemisch dessen Tetra-, Penta- und Hexamethyl-Derivate im Verhältnis von 2-98 Vol. -%, insbesondere 1,5-97,0 Vol.-% Tetramethyl-, 2-98 Vol. -%, insbesondere 1,5-97,0 Vol.-% Pentamethyl- und restlichem Hexamethyl-Derivat.

Die gestellte Aufgabe wird auch mittels eines immunmodulierenden, antiblastomatösen Heilmittels gelöst, das dadurch gekennzeichnet ist, dass es ein Gemisch aus Dextrin und pharmakologisch vertretbaren Derivaten des Tri-p-aminotriphenylchlormethans umfasst, z.B. in Form eines Gemisches aus Tetra- und Pentamethyl-Derivaten oder ein Gemisch aus Tetra- und Hexamethyl-Derivaten oder ein Gemisch aus Penta- und Hexamethyl-Derivaten bzw. ein Gemisch aus Tetra-, Penta- und Hexamethyl-Derivaten im Verhältnis von 10,0-95,5 Vol.-% des entsprechenden Derivats bzw. Derivatengemisches des Tri-p-aminotriphenylchlormethans und restlichem Dextrin.

Dabei wird das Gemisch aus Tetra- und -Derivaten des Tri-p-aminotriphenylchlomethyls im Verhältnis von 2,0-98,0 Vol.-% Tetramethyl-Derivat und restlichem Pentamethyl-Derivat, das Gemisch aus Tetra- und Hexamethyl-Derivaten im Verhältnis von 2,0-98,0 Vol.-% Tetramethyl-Derivat und restlichem Hexamethyl-Derivat, das Gemisch aus Penta- und Hexamethyl-Derivaten im Verhältnis von 2,0-98,0 Vol.-% Pentamethyl-Derivat und restlichem Hexamethyl-Derivat, das Gemisch aus Tetra-, Penta- und Hexamethyl-Derivaten im Verhältnis von 2-98 Vol. -%, insbesondere 1,5-97,0 Vol.-% Tetramethyl-Derivat, 2-98 Vol.-%, insbesondere 1,5-97,0 Vol.-% Pentamethyl-Derivat und restlichem Hexamethyl-Derivat gewählt.

Das Heilmittel mit immunmodulierender und antiblastomatöser Wirkung enthält ein pharmakologisch vertretbares Tetra- oder Penta- bzw. Hexamethyl-Derivat des Tri-p-aminotriphenylchlormethans oder deren Gemische bzw. Gemisch aus einem Derivat und Dextrin oder Gemische aus diesen Derivaten und Dextrin.

Das Tetramethyl-Derivat des Tri-p-aminotriphenylchlormethans stellt dabei Methylviolett 2B (Tetramehtylpararosaniliniumchlorid, in der Strukturformel ist R₆ = NH₂, die Bezeichnungen für R₁, R₂, R₃, R₄, R₅ stimmen mit den oben genannten überein, die Summenformel ist C₂₃H₂₆N₃Cl, das Molargewicht beträgt 379,94) dar, das ein wasser- und alkohollösliches, feinkörniges, braunes, kristallines Pulver grünlichen Farbtons ist; das Pentamethyl-Derivat stellt Methylviolett 6B (Pentamethylpararosaniliniumchlorid, in der Strukturformel ist R₆ = NHCH₃, die Bezeichnungen für R₁, R₂, R₃, R₄, R₅ stimmen mit den oben genannten überein, die Summenformel ist C₂₄H₂₈N₃Cl, das Molargewicht beträgt 393,97) dar und ist ein homogenes, wasser- und alkohollösliches, feinkörniges, kristallines Pulver bronze-grünlicher Farbe; und das Hexamethyl-Derivat stellt Methylviolett 10B (Hexamethylpararosaniliniumchlorid, in der Strukturformel ist R₆ = N(CH₃)₂, die Bezeichnungen für R₁, R₂, R₃, R₄, R₅ stimmen mit den oben genannten überein, die Summenformel ist C₂₅H₃₀N₃Cl, das Molargewicht beträgt 407,99) dar und ist ein wasser- und alkohollösliches, grünes, kristallines Pulver.

Gemische aus Derivaten des Tri-p-aminotriphenylchlormethans und Gemische aus dessen Derivaten und Dextrin in den o.g. Verhältnissen stellen ein wasserlösliches, grünliches Pulver dar.

Derivate des Tri-p-aminotriphenylchlormethans in Form von deren Gemischen werden mit nachfolgender Trennung als Tetra-, Penta- und Hexamethyl-Derivate durch Methylieren von Parafuchsin mit Jodmethyl gewonnen (s. A.N. Nesmejanow, N.A. Nesmejanow, Grundlage organischer Chemie, Verlag Chemie, Moskau, 1974, Band 2, S. 200), wobei das Pentamethyl-Derivat (Basic Violet K) auch mittels Oxidation von Dimethylanilin mit Kupfersalzen (s. Schadstoffe in der Industrie, Teil 1, organische Stoffe, Verlag chemischer Literatur, Ausgabe 4, Leningrad, 1963, S. 639) und reines Hexamethyl-Derivat (Kristallviolett) durch Kondensation von Di-(p-Dimethylamino)-Benzophenon (Michlersches Keton) mit Dimethylanilin und nachfolgendem Sauermachen (s. A.N. Nesmejanow, N.A. Nesmejanow, Grundlage organischer Chemie, Verlag Chemie, Moskau, 1974, Band 2, S. 200) gewonnen werden können.

Das Zielprodukt als einsatzfertiges Heilmittel wird durch Reinigung der erhaltenen Gemische gewonnen, beispielsweise durch Umkristallisation sowie im Chromatographieverfahren mittels Durchlaufen von Wasserlösungen dieser Salze durch eine Sorptionsschicht, beispielsweise chromatographischer Zellstoff oder Bentonitlehm in der Trennsäule.

Dextrin wird mittels Erwärmung von trockener Stärke auf 200-250° C gewonnen (s. N.L. Glinka, Allgemeine Chemie, Verlag Chemie, 1978, S. 494).

Die benötigten Gemische werden durch Wahl bestimmter Substanzen in einem vorgegebenen Verhältnis gewonnen.

Das pharmakologisch vertretbare Derivate des Tri-p-aminotriphenylchlormethans enthaltende Heilmittel sowie Gemische aus Dextrin und den Derivaten haben eine antiblastomatöse Wirkung, die z.B. mit der vom Erfinder festgestellten und als disymmetrisch bezeichneten therapeutischen Wirkung begleitet wird, die bei unterschiedlichen Temperaturen und an unterschiedlichen Körperpunkten gemessen wird; diese Wirkung prägt sich beispielsweise in einem Temperaturbereich von 2-3° C in 0,5-2 Stunden nach der Heilmitteleinnahme aus und dauert 3-4 Stunden (s. M.W. Kutuschow, Krebs, Heilung ist möglich, Verlag Newa, St-Petersburg, 2003). Das Heilmittel weist auch eine anabolische Aktivität auf und fördert Zell- und Humoralschutzfaktoren des Organismus.

Dem Wirkungsmechanismus des pharmakologisch vertretbare Derivate von Tri-p-Aminotriphenylchlormethan enthaltenden Heilmittels liegt die lonenfähigkeit zugrunde, durch die eine Wechselwirkung mit Phospholipiden der Zellmembranen die Bilanz des ionischen trans-Membranpotentials zu stören, wodurch ein Zellsterben erfolgt. Die vom Erfinder durchgeführten Untersuchungen zeigten auch, dass das Heilmittel ein Inhibitor der Mitochondrienfermente von bösartigen Zellen ist und das Sterben von Mitochondrien der Karzinomzellen verursacht, wobei es die Aktivität der NADPH-Cytochrom-c-Reduktase unterdrückt, wodurch eine Energieblockierung erfolgt (s. Shumyantseva V.V., Uvarov V.Yu., Byakova O.E., Archakov A.I., Biochem. Molec. Biol. Intern, 1996, Nr.38, Seiten 829-838). Dabei wurde festgestellt, dass die Wirksamkeit des Heilmittels umso höher ist, je mehr sein Absorptionsspektrum in Richtung des UV-Bereichs verschoben wird. Ferner stellte sich heraus, dass Dextrin als eine der Substanzen die Toxitizität des Heilmittels wesentlich vermindert und somit den Dosisbereich erweitert.

Dies wird durch Labor- und Patientenuntersuchungen bestätigt, deren Blut auf immunologische Werte des Leukozyten- und Lymphsystems geprüft wurde.

Ausgeführt als technisches oder chemisch reines Salz bzw. hochreines oder medikamentenreines oder medikamentenhochreines Salz, kann das Heilmittel nach Pharmakopöeforderungen an die Arzneimittel (s. Gesetz der Russischen Föderation über Heilmittel Nr. 86-FZ vom 22.06.98 sowie die Branchennorm OST 91500.05.001,00, Qualitätsnormen der Heilmittel, Bestimmungen, Publikation in der Rossijskaja Gaseta vom 28.11.2001) dem Patienten unter Bezug des Reinigungsgrads des Präparats in einer isotonischen Natriumchloridlösung intravenös und peroral in Kapseln eingeführt sowie als Substanz in Salben oder als Arzneiklistiere bzw. Zäpfchen eingesetzt werden, wobei der Einsatz des Heilmittels keine allergischen oder sonstigen Nebenwirkungen verursacht und seine Wirksamkeit (bei Behandlung bestimmter Krankheiten) in einigen Fällen höher als die des Prototyps ist.

Untersuchungsergebnisse des Wirkungsmechanismus des Arzneimittels gemäß der vorliegenden Erfindung werden in den folgenden Beispielen 1-3 ausgeführt, und der mögliche Einsatz des Arzneimittels wird durch die folgenden Beispiele 4-10 bestätigt.

### Beispiel 1

In Prüfgläser (14 Prüfgläser je 10ml) der ersten Gruppe mit human malignant melanoma A 375 in Pufferlösung wurde das Arzneimittel in einer Verdünnung von 10⁻⁷mmol/l in Form von Methylviolett 2B (Tetramethyl-Derivat des Tri-p-aminotriphenylchlormethans), Methylviolett 6B (Pentamethyl-Derivat des Tri-p-aminotriphenylchlormethans), Methylviolett 10B (Hexamethyl-Derivat des Tri-p-aminotriphenylchlormethans), Gemischen aus Methylviolett 2B und Methylviolett 6B, Methylviolett 2B und Methyl- violett 10B, Methylviolett 6B und Methylviolett 10B, Gemischen aus Methylviolett 2B, Methylviolett 6B und Methylviolett 10B (im gleichen Verhältnis der Substanzen), Gemischen aus Dextrin und deren Salze sowie Gemischen aus Dextrin und den angegebenen Gemischen dieser Salze (im Verhältnis von 50 Vol.-%, Dextrin und restlichem Salz oder restlichem Salzgemisch (im angegebenen Verhältnis) eingeführt. Dasselbe wurde mit einer Verdünnung von 10⁻⁹mol/l (14 Prüfgläser je 10 ml) ausgeführt.

In Prüfgläser der zweiten Gruppe mit HFF (human forskin fibroblast) wurde das Heilmittel in denselben Zusammensetzungen und Verdünnungen eingeführt.

Ferner wurde eine dritte Kontrollgruppe der Prüfgläser (6 Stück) mit demselben Krebszellenpool und Fibroblasten, in die Adriamycin und Zyclophosphan in derselben Verdünnung eingeführt wurden, sowie eine entsprechende Prüfgläsergruppe mit physiologischer Kochsalzlösung gebildet.

Nach einer Thermostatisierung der o.g. Prüfgläser im Laufe einer Woche bei 37° C (in der Dunkelheit) wurden die Inhalte analysiert.

Die gewonnenen Ergebnisse zeigten:
In der Versuchsgruppe mit dem Heilmittel in einer Verdünnung von 10⁻⁷mmol/l unterdrückt das Heilmittel die Fortpflanzung der Melanomzellen um 86-93 % im Vergleich zur Fortpflanzung dieser Zellen im Prüfglas mit physiologischer Kochsalzlösung und zur Fortpflanzung der Fibroblasten um 25-27 %, wobei das Erscheinungsbild für alle Zusammensetzungen des Heilmittels ungefähr gleich ist.

In Prüfgläsern der Kontrollgruppe mit Adriamycin wurde eine Fortpflanzung der Melanomzellen um 42 % und mit Cyclophosphan um 55 % vermindert.

In der Versuchsgruppe mit dem Heilmittel in einer Verdünnung von 10⁻⁹ mmol/l unterdrückt das Heilmittel die Fortpflanzung der Melanomzellen um 75-90 % im Vergleich zur Fortpflanzung dieser Zellen in physiologischer Kochsalzlösung und die Fortpflanzung der Fibroblasten um 5-10 %, wobei in Prüfgläsern der Kontrollgruppe mit Adriamycin die Fortpflanzung der Melanomzellen um 30 % und mit Cyclophosphan um 35 % vermindert wurde.

Die gewonnenen Daten weisen nach, dass das angebotene Heilmittel selbst in sehr starken Verdünnungen eine ausgeprägte antiblastomatöse Wirkung und höhere Aktivität aufweist als bekannte antiblastomatöse Präparate und durch eine geringe zytotoxische Wirkung gegen Fibroblasten gekennzeichnet ist.

### Beispiel 2

In Prüfgläser (50 Prüfgläser je 10ml) der Versuchsgruppe mit "glatten" Mikrosomen der Krebszellen MCF-7 (Brustdrüsenadenokarzinom) in einer Pufferlösung wurde das Arzneimittel in einer Verdünnung von 10⁻⁵ mmol/l in Form von Gemischen aus Methylviolett 2B (Tetramethyl-Derivat des Tri-p-aminotriphenylchlormethans) und Methylviolett 6B (Pentamethyl-Derivat des Tri-p-aminotriphenylchlormethans) in zwei Verhältnissen: 98,0 Vol.-% Methylviolett und restliches Methylviolett 6B sowie 98,0 Vol.-% Methylviolett 6B und restliches Methylviolett 2B, Methylviolett 2B und Methylviolett 10B (Hexamethyl-Derivat des Tri-p-aminotriphenylchlormethans) in 2 Verhältnissen: 98 Vol.-% Methylviolett 2B und restliches Methylviolett 10B, aus Methylviolett 6B und Methylviolett 10B in zwei Verhältnissen: 98,0 Vol.-% Methylviolett 6B und restliches Methyl-violett 10B sowie 98,0 Methylviolett 10B und restliches Methylviolett 6B, Gemischen aus Methylviolett 2B, Methylviolett 6B und Methylviolett 10B in vier Verhältnissen: 1.) 2,0 Vol.-% Methylviolett 2B, 50,0 Vol.-% Methylviolett 6B und restliches Methylviolett 10B, 2.) 50,0 Vol.-% Methylviolett 2B, 2,0 Vol.-% Methylviolett 6B und restliches Methylviolett 10B, 3.) 50,0 Vol.-% Methylviolett 6B, 2,0 Vol.-% Methylviolett 10B und restliches Methylviolett 2B, 4.) 20,0 Vol.-% Methylviolett 2B, 25,0 Vol.-% Methylviolett 6B und restliches Methylviolett 10B und Gemischen aus Dextrin und Salzen sowie den angegebenen Gemischen dieser Salze (in den o.g. Zusammensetzungen) im Verhältnis: 10,0, 25,0, 75,0, 90,0 Vol.-% Dextrin und restliches Salz bzw. Salzgemisch eingeführt. Dasselbe wurde auch mit einer Verdünnung von 10⁻⁹mmol/l ausgeführt.

In Prüfgläser (10ml) der zweiten Gruppe mit Mikrosomen von Rattenleber wurde das Heilmittel in denselben Zusammensetzungen und Verdünnungen eingeführt.

Ferner wurde eine dritte (Kontroll-)Gruppe der Prüfgläser (6 Stück) mit Mikrosomen desselben Krebszellenpools und Rattenleber, in die Adriamycin und Zyclophosphan in derselben Verdünnung eingeführt wurden, sowie eine entsprechende Prüfgläsergruppe mit physiologischer Kochsalzlösung gebildet.

In den Prüfgläsern der Gruppen wurde die Konzentration von Zytochrom C bestimmt, die sich auf 50 µM für Krebsmikrosome und 20 µM für Mikrosome der Rattenleber beläuft.

Nach der Thermostatisierung der o. g. Prüfgläser im Laufe von 20 min bei 25° C (in der Dunkelheit) wurden die Inhalte analysiert.

Die gewonnenen Ergebnisse zeigten:
In der Versuchsgruppe wurde die Konzentration von Zytochrom C in den Prüfgläsern mit den eingeführten Salzgemischen im Bereich bis 60 µM und in den Prüfgläsern mit den eingeführten Gemischen aus Salzen und Dextrin im Bereich bis 65 µM bestimmt.

In den Prüfgläsern mit Adriamycin und Zyclophosphan weist Konzentration von Zytochrom C 40-45 µM auf. Im Prüfglas mit der physiologischen Kochsalzlösung wurden keine Änderungen in der Konzentration von Zytochrom C beobachtet. Lebermikrosome der Ratten wurden von den Präparaten nicht beeinflusst. Die Konzentration von Zytochrom C in allen Prüfgläsern blieb im Bereich von 19-20 µM.

Die gewonnenen Ergebnisse führen zu der Annahme, dass das Heilmittel das Freisetzen des "aggressiven" Proteins von Zytochrom C aus den Membranen der Krebszellenmitochondrien fördert, das eine Apoptose dieser Zellen verursacht, wobei es einen Zerstörungsmechanismus der DNS durch Kaspasen auslöst (s. Wilson B.E., Mochon E.A., Boxer L.M., Induction of blc-2 expression by phosphorylated CREB proteins during B-cell activation and rescue from apoptosis, Mol. Cell. Biol., 1996, V. 16, Seiten 5546-5556).

### Beispiel 3

Bei den Mäusen der Rasse B-57 wurde eine Reaktion auf das Arzneimittel in Form von Salzen aus Methylviolett 2B, Methylviolett 6B und Methylviolett 10B sowie Gemischen dieser Salze mit Dextrin in zwei Verhältnissen geprüft: 5,0 Vol.-%. Dextrin und restliches Salz sowie 95,0 Vol.-% Dextrin und restliches Salz.

Die Versuchsgruppe umfasste 60 Mäuse und wurde in 6 Untergruppen zu je 10 Mäusen eingeteilt. Die Kontrollgruppe umfasste 10 Mäuse; die Untergruppen der Versuchsgruppe wurden dabei von der Kontrollgruppe getrennt gehalten, und Mäuse jeder Untergruppe wurden von 1 bis 10 mit einem Farbmittel, in diesem Fall mit Viride nitens, gekennzeichnet.

Die Versuchsgruppe erhielt das Arzneimittel im Laufe von 10 Tagen: die erste Untergruppe das Gemisch aus 2,0 Vol.-% Dextrin und restlichem Methylviolett 2B, die zweite Untergruppe das Gemisch aus 90,0 Vol.-% Dextrin und restlichem Methylviolett 2B, die dritte Untergruppe das Gemisch aus 5,0 Vol.-% Dextrin und restlichem Methyl- violett 6B, die vierte Untergruppe das Gemisch aus 90,0 Vol.-% Dextrin und restlichem Methylviolett 6B, die fünfte Untergruppe das Gemisch aus 5,0 Vol.-% Dextrin und restlichem Mehtylviolett 10B, die sechste Untergruppe das Gemisch aus 90,0 Vol.-% Dextrin und restlichem Methylviolett 10B. In jeder Untergruppe erhielten die Mäuse Nr. 1 bis 8 das Präparat als Getränk (in Wasserlösung) und die Mäuse Nr. 9-10 als Injektion in das Bauchfell. Dabei betrug die Einmaldosis für die Mäuse Nr. 1-2 2 mg, für Nr. 3-4 5 mg, für Nr. 5-6 10 mg, für Nr. 7-8 20 mg und Nr. 9-10 15 mg pro 1 ml Injektionswasser. Die Mäuse der Kontrollgruppe erhielten kein Präparat.

Ergebnisse: nach einer Woche seit der Einführung des Arzneimittels beobachtete man keine Unterschiede im Verhalten der geraden Untergruppen und der Kontrollgruppe. Die Tiere Nr. 5-6 der ungeraden Untergruppen sind wenig beweglich, Tiere Nr. 7-8 der ungeraden Untergruppen starben.

Die Mäuse mit ungeraden Nummern in allen Untergruppen wurden am 10. Tag und Mäuse mit geraden Nummern am 21. Tag getötet.

Die Spuren des Arzneimittels wurden in den Nieren der Nr. 3-6 der ungeraden Untergruppen und bei Nr. 5-8 der geraden Untergruppen festgestellt.

Bei einer histologischen Prüfung wurde ein minimaler Einfluss des Gemisches mit Methylviolett 10B (als B.V.2 bezeichnet) und ein maximaler Einfluss des Gemisches mit Methylviolett 2B festgestellt.

Daraus kann man unter Berücksichtigung der Massenverhältnisse schließen, dass die Einmaldosis 2,0 g für den menschlichen Organismus vertretbar ist. Genauere Daten können aber erst nach entsprechenden Untersuchungen geliefert werden.

### Beispiel 4

Den Mäusen der Rasse B-57 der Versuchs- und Kontrollgruppe (je 60 Mäuse) wurde Melanom-16 eingeimpft. Die Versuchsgruppe wurde in 6 Untergruppen und die Kontrollgruppe in 3 Untergruppen mit je 10 Mäusen eingeteilt. Jede Untergruppe der Versuchsgruppe erhielt 5 mg Arzneimittel als Wasserlösung von Methylviolett 2B, Methylviolett 6B und Methylviolett 10B sowie Gemischen dieser Salze mit Dextrin im Verhältnis von 25:75 Vol.-%.

Die erste und zweite Untergruppe der Kontrollgruppe erhielt als Getränk eine Wasserlösung von Adriamycin und Zyclophosphan in einer Dosis von 5 mg; die dritte Untergruppe erhielt keine Präparate.

Ergebnisse: Am 39. Tag des Versuchs sind 39 von 60 Mäusen der Versuchsgruppe am Leben geblieben. 11 Mäuse (4 aus der ersten, je 3 aus der zweiten und dritten und eine Maus aus der vierten Untergruppe) starben am 19. Tag; 5 Mäuse (je 2 aus der ersten und zweiten und eine aus der dritten Untergruppe) starben am 23. Tag und 5 Mäuse (2 aus der ersten und je eine aus der zweiten, vierten und fünften Untergruppe) am 34.Tag des Versuchs. Siebzehn überlebende Mäuse (eine aus der ersten, 3 aus der zweiten, eine aus der dritten, je 4 aus der vierten und fünften und 3 aus der sechsten Untergruppe) hatten Blastome von ca. 0,2 x 0,2 mm; bei der Sichtprüfung der restlichen Mäuse wurden keine Blastome festgestellt. Die überlebenden Mäuse wurden am 36. Tag des Versuchs getötet. Während der Sektion stellte man bei 9 Mäusen (eine aus der ersten, 4 aus der vierten und je 2 aus der fünften und sechsten Untergruppe) vereinzelte, pulmonale Metastasen und bei 4 von diesen 9 Mäusen (eine aus der ersten, 2 aus der zweiten, eine aus der fünften Untergruppe) auch hepatische Metastasen fest. Bei den anderen Mäusen wurden keine Metastasen und keine sichtbare Änderungen innerer Organe festgestellt.

Die erste und zweite Untergruppe der Kontrollgruppe: bis zum 36. Tag des Versuchs überlebten 2 Mäuse der ersten und 5 Mäuse der zweiten Untergruppe. Die Blastome hatten durchschnittliche Abmessungen von 1,5 x 1,5 cm. Während der Sektion stellte man Lungen- und Leberverletzungen fest.

Alle Mäuse aus der dritten Untergruppe starben am 10., 12., 20. und 25. Tag. Bei der Untersuchung stellte man blutende Rückenblastome mit einer Größe von durchschnittlich 2,5 x 2,0 cm fest. Durch eine hystologische Untersuchung wurde eine totale Lungenlysis festgestellt.

Schlussfolgerungen: das Arzneimittel besitzt eine antiblastomatöse Wirkung und verursacht keine Nebenwirkungen.

### Beispiel 5

Bei einem Hund von 3 Jahren und mit einem Gewicht von 16 kg wurde ein Non-Hodgin-Lymphom (NHL) diagnostiziert. Der Zustand war schwer, Atemnot und Kachexie. Im Halsbereich befanden sich Geschwülste mit einer Größe von 7 x 5 cm rechts und 11 x 10 cm links. Auf dem Röntgenbild vom 20.10.99 waren mehrere geschwulstähnliche Ausbildungen im Mittelfell zu erkennen.

Die Behandlung mit dem Arzneimittel wurde am 30.10.99 begonnen. Dem Hund wurden 500 mg Methylviolett 2B pro 500 ml physiologischer Kochsalzlösung tropfenweise intravenös eingeführt. Nach drei Tagen wurde ein Gemisch aus Methylviolett 2B und Methylviolett 6B (je 250 mg) pro 500 ml physiologischer Kochsalzlösung intravenös eingeführt sowie eine perorale Einnahme eines Gemisches aus Dextrin, Methylviolett 2B, Methylviolett 6B und Methylviolett 10B (je 50 mg von jeder Substanz in einer Einmaldosis von 200 mg) als Pulver zweimal am Tag veranlasst. Es folgten wiederholte intravenöse Einführungen des Gemisches aus Methylviolett 2B und Methylviolett 6B sowie eine perorale Einnahme des o.g. Gemisches mit Dextrin nach einer Woche.

Zwei Wochen nach Beginn der Behandlung wurde eine Verbesserung des Allgemeinzustands und der biochemischen und klinischen Blutwerte beobachtet. Nach drei Wochen wurden sichtbare Geschwülste um den Faktor 2,5 kleiner.

Die Behandlung mit Methylviolett 2B und Methylviolett 6B umfasste eine wöchentliche intravenöse Einführung des Gemisches 3 Monate lang; die perorale Einnahme des o.g. Gemisches (mit Dextrin) verminderte sich auf einmal täglich.

Bei einer Untersuchung nach der Behandlung wies der Hund einen guten Zustand auf, das Gewicht betrug 19,5 kg, und Geschwülste am Hals wurden nicht gefunden Der Blutbefund war ohne Pathologie. Auf dem Röntgenbild vom 02.02.2000 waren vereinzelte geringe Lungenmetastasen zu erkennen.

### Beispiel 6

Bei einem Hund von 6 Jahren und einem Gewicht von 21 kg wurden ein Sarkom des rechten Unterarmes und pulmonale Metastasen diagnostiziert. Auf dem Röntgenbild vom 10.07.2002 war eine Zerstörung der Knochenhaut und der 1.-7. rechten Rippen, mehrere pulmonale Metastasen rechts und links sowie eine Geschwulst mit einer Größe von 10x15x12 cm sichtbar.

Am 12.07.02 wurde die Behandlung begonnen: jeden 2. Tag wurde eine Dosis aus 50 mg Methylviolett 10B (als B.V.2 bezeichnet), verdünnt mit 9 % NaCl und gelöst in 250 ml Wasser in einer Menge von 2 ml intravenös verabreicht; tägliche Salbenverbände um die Geschwulst (2 % Methylviolett 10B und eine restliche Kinderpflege-Creme) wurden vorgenommen.

Drei Wochen nach Beginn der Behandlung hatten die Geschwulstabmessungen eine Größe von 7x7x3 cm. Der Zustand war stabil und nach weiteren zwei Wochen befriedigend; auf dem Röntgenbild waren eine verkapselte Geschwulst mit einer Größe von 2x2,5x1 cm und Spuren von Rippen- und Lungenmetastasen sichtbar.

### Beispiel 7

Bei einer Kranken I. von 35 Jahren, die den Arzt im November 2002 besuchte, wurde 2001 eine Exstirpation der Gebärmutter und des -anhangs wegen Krebses des rechten Eierstocks durchgeführt. Das Tomogramm vom 28.08.02 zeigte ein Rezidiv, eine Geschwulst mit einer Größe von 8,8 x 9, 8 x 14 cm mit einer Durchwachsung der Harnblase und des Rektums.

Die Kranke wurde mit einem Klistieren des Mastdarms behandelt: im Laufe von fünf Tagen einmal täglich, nach fünf Prozeduren einmal pro zwei Tage und danach 15 Klistiere jeden dritten Tag. Sie wurde mit einer Einmaldosis von 20 mg Arzneimittel (B.V.2) pro 15-20 ml Wasser, verdünnt in 2 ml 70%-igem Ethylalkohol, behandelt.

Nach 2 Wochen der Behandlung wies die Geschwulst bei einer Ultraschalluntersuchung eine Größe von 3,8 x 4, 2 x 3,1 cm mit ebenen Konturen auf; die Geschwulst war von der Wand des Mastdarms abgegangen, und die Verformung der Harnblasenwand war gering.

Nach der Kur war die Geschwulst gemäß einer Ultraschalluntersuchung verkapselt und wies eine Größe von 1,3 x 2, 0 x 1,6 cm auf; die Kapselwände hatten eine Stärke von 0,5 cm, und es gab keine Beschwerden. Es wurde die Fortsetzung der Behandlung mit Kurpausen (1,0-1,5 Monate) empfohlen. Bei einer Kontrolluntersuchung mit Ultraschall am 02.03.2004 wurde die Geschwulst nicht mehr gefunden.

Die Blutwerte vor der Behandlung waren: Leukozyten bis 10.000, Erythrozyten 2,5 Mio., Thrombozyten 110.000. Die Blutwerte nach Behandlungskur betrugen: Leukozyten 9.000, Erythrozyten 5,5 Mio., Thrombozyten 300.000.

### Beispiel 8

Bei einem kranken R. von 62 Jahren wurde ein papillärer Schilddrüsenkrebs diagnostiziert. Vor der Behandlung wies die Geschwulst gemäß einer Ultraschalluntersuchung im rechten Lappen eine Größe von 5x3x1,7 cm auf.

Der Kranke wurde mit dem Arzneimittel (B.V.2) 2 Wochen lang behandelt: zweimal täglich je eine Gelatinekapsel mit 20 mg Arzneimittel peroral nach dem Essen sowie Klistiere in einer Einmaldosis von 100 ml Lösung, bestehend aus 50 mg Arzneimittelpulver, verdünnt in 2 ml 70%-igem Ethylalkohol und gelöst in 100 ml Wasserlösung mit 0,9% NaCl.

Im Laufe von 7 Tagen wurde bei dem Kranken 3 Stunden nach der Einnahme des Arzneimittels eine dissimmetrische Wirkung beobachtet, die sich durch plötzliche Fieberschauer und unterschiedliche Temperaturen der rechten und linken Körperhälfte, und zwar von 35,4-36,9° C der rechten und 37,3-38,6° C der linken, im Laufe von 1,0-1,5 Stunden äußerte. Mit einer weiteren Einnahme des Präparats verschwanden die o. g. Temperaturunterschiede praktisch vollständig.

Nach der Kur zeigte eine Ultraschalluntersuchung eine primäre Geschwulst und keine Metastasen. Bei einer Kontrolluntersuchung nach 2 Monaten war der Zustand befriedigend, und es gab keine Beschwerden.

### Beispiel 9

Bei einer kranken Sch. von 18 Jahren wurde im September 2002 ein Tumor der rechten Brustdrüse mit einer Größe von 2x2,5x2 cm festgestellt. Auf dem Mammogramm war eine fibrozystische Mastopathie der rechten Brustdrüse zu erkennen. Eine Biopsieprobe zeigte atypische Zellen.

Es wurden zwei- bis dreimalige Anstriche im Bereich der Geschwulst mit einem Gemisch aus Pulver des B.V.2 und einer Kinderpflege-Creme im Verhältnis von 10:90 % verordnet. Wiederholte Biopsieproben nach 1,5 Monaten zeigten keine atypische Zellen. Die Geschwulst war praktisch nicht mehr ertastbar.

### Beispiel 10

Bei einem Kranken Ju. von 48 Jahren wurde ein Adenokarzinom des Enddarms mit Lebermetastasen diagnostiziert. Im April 1999 wurde ein Kolon herausgeschnitten und ein Kolostoma angelegt. Eine prophylaktische Chemotherapie war nicht wirksam (erneute Schmerzen im Bauchbereich, periodisches Erbrechen, allgemeine Schwäche). Im August 2002 suchte der Kranke wegen dieser Beschwerden den Arzt auf. Ein Tomogramm vom 21.09.02 zeigte im Milzeckenbereich eine geschwulstähnliche Bildung mit einer Größe von 7,0 x 5,5 cm, unebenen Konturen und Metastasen im rechten Leberlappen.

Es wurde folgende Behandlung durchgeführt: 10 Tage lang die perorale Einnahme eines mit 100 ml Wasser verdünnten Gemisches aus Dextrin und Methylviolett 2B, Methylviolett 6B und Methylviolett 10B (B.V.2) (in einem entsprechenden Verhältnis von 25:30:30:15) je 1,0 g zweimal täglich 30 Minuten vor dem Essen sowie morgens und abends 0,5g B.V.2, gelöst in 50ml Wasser, in das Kolostoma. Darüber hinaus erfolgte mit Einverständnis des Kranken eine intravenöse Einführung von 500 ml einer 1%-igen Lösung des Präparats zur Wirkungssteigerung. Die Kur dauerte 3 Monate. Nach der Kur ist der Zustand befriedigend. Das Tomogramm zeigte keine Geschwülste in der Bauchhöhle und keine hepatische Metastasen. Die Blutwerte waren in Ordnung.

### Beispiel 11

Bei einer Kranken N. von 25 Jahren wurden bei einem Arztbesuch am 23.07.1999 Beschwerden von Alopecia totalis, eine periodische Schwäche und eine Depression festgestellt.

Eine Alopezie begann mit Haarausfall der Brauen und Wimpern vor 6 Jahren nach einer Grippe, und dann erschienen nach 6 Monaten Haarausfallherde am Kopf. 2 Jahre lang wurde die Kranke mit Hormonen sowie mit Salben lokal behandelt. Bei einer Anamnese wurde festgestellt, dass die Kranke im Alter von 7 Jahren eine akute Respirationskrankheit und eine Woche danach Windpocken gehabt hatte, wodurch wahrscheinlich eine latente Autoimmunkrankheit verursacht wurde. Es wurde eine Autoimmunkrankheit mit komplizierter Alopecia totalis diagnostiziert. Blutuntersuchungen auf zelluläre und humorale Immunität bestätigten die Diagnose.

Zwei Kuren wurden unter Einsatz des Arzneimittels durchgeführt. Die erste Kur bestand in der Einnahme eines Gemisches aus Dextrin, Methylviolett 2B, Methylviolett 6B und Methylviolett 10B im Verhältnis 25:25:25:25 nach dem Schema 0,2 ml 1%-ige Wasserlösung zweimal täglich 30 Minuten vor dem Essen im Laufe von 3 Monaten.

Nach 3 Wochen der Behandlung erschienen nagende Schmerzen in der Nierenprojektion. Dies wurde als Reaktion der Nebennieren auf Unterdrückung fixierter, hyperaktiver Mikrophagen durch das Präparat angenommen. Ein Tomogramm der Bauchhöhle, Nieren und Nebennieren zeigte, dass die linke und rechte Nebenniere etwas größere Abmessungen und Verformungen aufwiesen. Die Behandlung wurde fortgesetzt und nach einer Woche waren die Schmerzen in der Nierenprojektion verschwunden. Nach 2 Monaten Behandlung erschienen Wollhaare auf dem Kopf und an den Beinen.

Nach der ersten Kur wurde die Behandlung für 2 Monate unterbrochen und dann eine zusätzliche Kur von 2 Monaten durchgeführt, die in einer Einnahme von Methylviolett 10B (B.V.2) mit einer Menge von 0,5 ml in einer 1 %-igen Wasserlösung zweimal täglich 30 Minuten vor dem Essen bestand.

Nach einem Jahr seit Beginn der Behandlung wiesen die Kopfhaare eine typische Dicke und Struktur auf. Zur Zeit wird kein Haarausfall beobachtet. Das Kontrolltomogramm vom 12.03.03 zeigte, dass die Nebennieren nicht vergrößert waren und eine dreieckige Form aufwiesen. Die Blutwerte waren in Ordnung.

Die Untersuchungsergebnisse in Bezug auf die zelluläre und humorale Immunität zeigt die folgende Tabelle:

| | Vor Behandlung | nach 4 Wochen | nach 6 Monaten |
|---|---|---|---|
| Immunoglobulin A | 60 | 170 | 249 |
| Immunoglobulin M | 50 | 98 | 190 |
| Immunoglobulin G | 800 | 1400 | 1750 |
| T-Limphozyten, % | 34,0 | 65,0 | 74,0 |
| B-Limphozyten, % | 10,0 | 14,0 | 16,0 |
| Phagozytose von Latexpartikeln, % | 43,0 | 60,0 | 67,3 |
| TNF | 15,6 | 23,0 | 32,5 |
| Helper-Zellen,% | 26,0 | 30,0 | 38,0 |
| Suppressor-Zellen, % | 12,0 | 18,0 | 17,0 |
| Killer-Zellen, % | 16,0 | 19,0 | 19,0 |

## Patentansprüche

1. Arzneimittel, das eine antiblastomatöse und immunmodulierende Wirkung hat, wobei es pharmakologisch vertretbare Derivate von Tri-p-aminotriphenylchlormethan enthält und folgende Struktur aufweist: 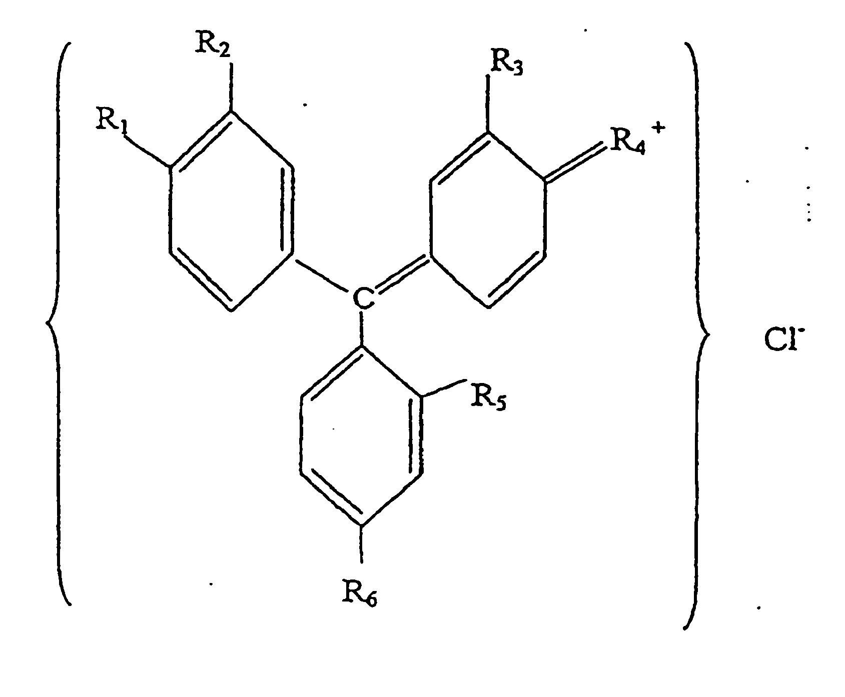 wobei R₁ = N(CH₃)₂, R₂ = H, R₃ = H, R₄ = N(CH₃)₂, R₅ = H und R₆ ist entweder NH₂, oder NHCH₃ oder N(CH₃)₂,
**dadurch gekennzeichnet, dass**
es ein Gemisch von 2-98 Vol.-% einer Verbindung mit R₆ = NH₂ und als Rest eine Verbindung mit R₆ = NHCH₃ ist oder
es ein Gemisch von 2-98 Vol.-% einer Verbindung mit R₆ = NH₂ und als Rest eine Verbindung mit R₆ = N(CH₃)₂ ist oder
es ein Gemisch von 2-98 Vol.-% einer Verbindung mit R₆ = NHCH₃ und als Rest eine Verbindung mit R₆ = N(CH₃)₂ ist oder
es ein Gemisch von 2-98 Vol.% einer Verbindung mit R₆ = NH₂, 2-98 Vol.-% einer Verbindung mit R₆ = NHCH₃ und als Rest eine Verbindung mit R₆ = N(CH₃)₂ ist.

2. Arzneimittel nach Anspruch 1, wobei es das Gemisch in einer Menge von 10-95 Vol.-% und weiterhin als Rest Dextrin enthält.

## Claims

1. A medicinal agent which has an antiblastomatous and immune-modulating effect, contains pharmacologically appropriate derivatives of tri-p-aminotriphenylchloromethane, and has the following structure: 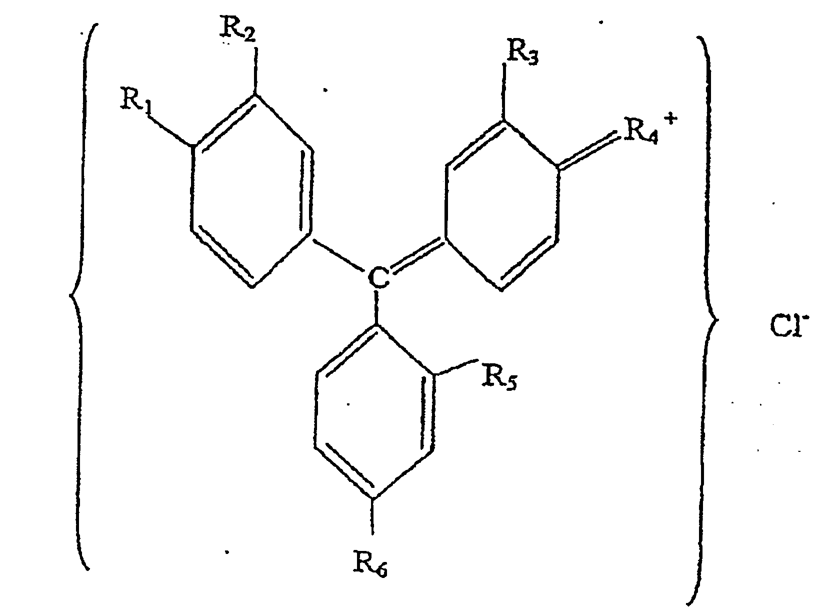 in which R₁ = N(CH₃)₂, R₂ = H, R₃ = H, R₄ = N(CH₃)₂, R₅ = H, and R₆ is either NH₂ or NHCH₃ or N(CH₃)₂,
**characterized in that**
it is a mixture of 2-98 vol.% of a compound in which R₆ = NH₂ and as a remainder, a compound with R₆ = NHCH₃, or
it is a mixture of 2-98 vol.% of a compound in which R₆ = NH₂ and as a remainder, a compound with R₆ = N(CH₃)₂, or
it is a mixture of 2-98 vol.% of a compound in which R₆ = NHCH₃ and as a remainder, a compound with R₆ = N(CH₃)₂, or
it is a mixture of 2-98 vol.% of a compound in which R₆ = NH₂, 2-98 vol.% of a compound in which R₆ = NHCH₃, and as a remainder, a compound with R₆ = N(CH₃)₂.

2. The medicinal agent as defined by claim 1, wherein it contains the mixture in a quantity of from 10-95 vol.% and contains dextrin as a remainder.

## Revendications

1. Médicament qui possède un effet anti-blastomateux et immunomodulateur, qui contient des dérivés de tri-p-aminotriphénylchlorométhane compatibles sur le plan pharmacologique et présente la structure suivante : 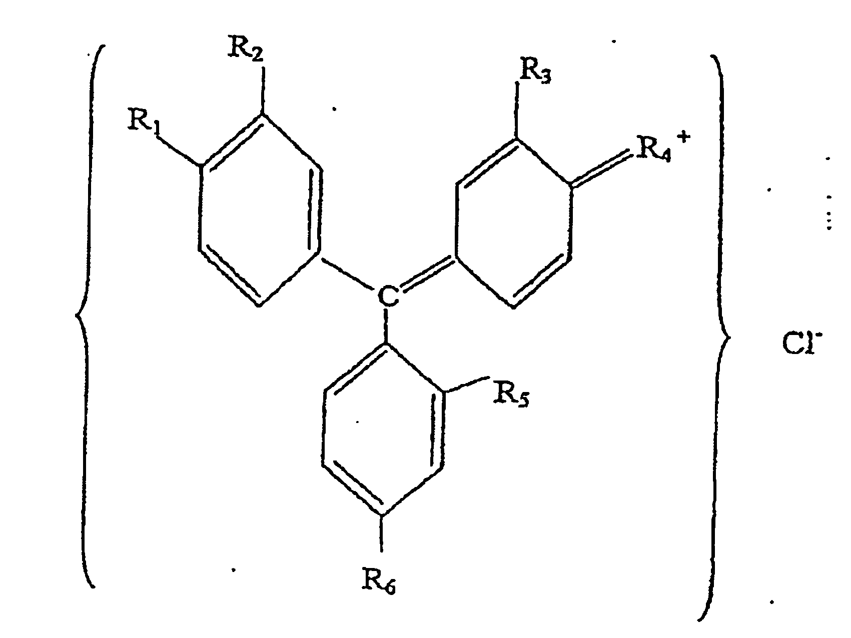 dans laquelle R₁ = N(CH₃)₂, R₂ = H, R₃ = H, R₄ = N(CH₃)₂, R₅ = H et R₆ est NH₂ ou NHCH₃ ou N(CH₃)₂,
**caractérisé en ce qu'**il s'agit
d'un mélange de 2 à 98 % en volume d'un composé ayant R₆ = NH₂ et pour le reste d'un composé ayant R₆ = NHCH₃, ou
d'un mélange de 2 à 98 % en volume d'un composé ayant R₆ = NH₂ et pour le reste d'un composé ayant R₆ = N(CH₃)₂ ou
d'un mélange de 2 à 98 % en volume d'un composé ayant R₆ = NHCH₃ et pour le reste d'un composé ayant R₆ = N(CH₃)₂ ou
d'un mélange de 2 à 98 % en volume d'un composé ayant R₆ = NH₂, de 2 à 98 % en volume d'un composé ayant R₆ = NHCH₃ et pour le reste d'un composé ayant R₆ = N(CH₃)₂.

2. Médicament selon la revendication 1, qui contient le mélange dans une quantité de 10 à 95 % en volume et contient pour le reste de la dextrine.
